# EUROPEAN PATENT APPLICATION

(11) **EP 2 202 305 A1**
(43) Date of publication of application: **30.06.2010**
(21) Application number: 08835360.2
(22) Date of filing: 01.10.2008
(51) Int. Cl.: C12N 15/09, C12Q 1/68

(54) **PRIMER FOR AMPLIFICATION OF RRNA OF BACTERIUM BELONGING TO THE GENUS LEGIONELLA, DETECTION METHOD, AND DETECTION KIT**

(30) Priority: 04.10.2007 JP 2007260596
(71) Applicant: Tosoh Corporation, Minato-ku Tokyo 105-8623 (JP); Intec Systems Institute, Incorporated, Koto-ku Tokyo 136-0075 (JP)
(72) Inventor: UNE, Kurando, Yokohama-shi Kanagawa 240-0022 (JP); SAITO, Juichi, Yamato-shi Kanagawa 242-0028 (JP); HAYASHI, Toshinori, Sagamihara-shi Kanagawa 228-0804 (JP); TAKIZAWA, Fumihide, Tokyo 136-0072 (JP); HASHIMOTO, Yoichi, Tokyo 155-0033 (JP)
(74) Representative: Kügele, Bernhard
(86) International application number: PCT/JP2008/067831
(87) International publication number: WO 2009/044773

(57) **Abstract**

[PROBLEMS] To detect a bacterium belonging to the genus *Legionella* rapidly, with high sensitivity, and specifically. [MEANS FOR SOLVING PROBLEMS] Disclosed are: a primer set for amplifying ribosomal RNA of a bacterium belonging to the genus *Legionella* specifically and highly efficiently; and a method for detecting ribosomal RNA of a bacterium belonging to the genus *Legionella* by using the primer set.

## Description

### FIELD OF THE INVENTION

The present invention relates to a primer set for amplification of rRNA of a bacterium belonging to the genus *Legionella,* a detection method, and a detection kit.

### DESCRIPTION OF THE RELATED ART

A bacterium belonging to the genus *Legionella* is a Gram-negative bacillus that is widely distributed in soil, fresh water, and the like in nature. A bacterium belonging to the genus *Legionella* is classified into about 50 species, and all the species of the bacterium have a potential to develop legionellosis. Nowadays, a bacterium belonging to the genus *Legionella* grows in artificial environments such as cooling towers, hot spring water circulation, and 24 h-running bath, etc. The infection with legionellosis by inhalation of the resulting aerosols has become a problem.

Legionellosis progresses fast and needs to be treated early. Penicillins and cephem antibiotics, which are used to treat other bacterial infections, are ineffective for legionellosis. Therefore, early diagnosis of legionellosis is necessary for appropriate treatment. In addition, a test to rapidly detect a bacterium belonging to the genus *Legionella* in an artificial environment is required from the perspective of legionellosis prevention. However, conventional testing methods for a bacterium belonging to the genus *Legionella* are a culture method. To obtain the test results requires a long time from five to seven days (see, Guidelines to prevent legionellosis supervised by Planning Division of Environmental Health Bureau in The Ministry of Health and Welfare, published by Building Mariagement and Education Center Foundation).

To solve this problem, immunological and genetic tests have been tried. Since the immunological test uses antibodies specific for *Legionella pneumophila,* the detection can be limited to *Legionella pneumophila* and has insufficient sensitivity to other species (see, "Kansenshougaku zasshi" (The Journal of Infectious Diseases) Vo. 71, No. 7, p634-643).

In addition, in the genetic test, the PCR method using 16S ribosomal DNA of a bacterium belonging to the genus *Legionella* by Jonas D. et al. is widely used. This method can detect a bacterium belonging to the genus *Legionella* with high sensitivity (Jonas D. et al., (1995) Journal of clinical microbiology, 33, 1247-1252).

On the other hand, as a method to measure RNA with high sensitivity, there is a method which amplifies RNA by RT-PCR method and measures the amplified product. However, in this case, the method generally requires a two-step process of reverse transcriptase (RT) and PCR processes, which can be not only an aggravating factor in reproducibility due to complicated operations, but also increase a risk of secondary contamination. The combination of these RT and PCR processes usually takes longer than two hours, and there is a problem in respect to the processing of multiple samples and the reduction of testing costs.

Widely used is a kinetic RT-PCR in which PCR process is performed under the presence of intercalating fluorescent dye to measure the increase in fluorescence over time. This method can conduct PCR process and a measurement process within 20 minutes. Unfortunately, the method also detects nonspecific amplified products such as a primer dimer. In addition, PCR method requires rapid change of the reaction temperature. This is a barrier for laborsaving and low-cost of reaction devices in automation. In addition, since RT-PCR method can also amplify DNA, DNase treatment and the like to completely remove DNA from the samples is required when the target is RNA as described above. The operation has been more complicated due to this process.

In contrast, as a method for amplifying only RNA at a constant temperature, NASBA method (see, Japanese Patent No. 2,650,159 and No. 3,152,927), TMA method (see, Japanese Patent No. 3,241,717), and the like have been reported. This RNA amplification method performs a chain reaction, comprising synthesizing double-stranded DNA containing a promoter sequence by a primer containing a promoter sequence for the target RNA, reverse transcriptase, if appropriate, and ribonuclease H (RNaseH); producing RNA containing a specific nucleic acid sequence derived from the target RNA by RNA polymerase using the double-stranded DNA as a template, and subsequently; becoming the RNA template for a synthesis of the double-stranded DNA containing the promoter sequence. After the RNA amplification, the amplified RNA is then detected by electrophoresis or a hybridization method using a nucleic acid probe that conjugates a detectable label.

Since these RNA amplification methods use a constant temperature and one step process for amplifying only RNA, these are suitable for simple measurement of RNA. However, the detection by the hybridization method and the like requires a complicated procedure. For this complication, the detection is not only unsuited for the processing of multiple samples and automated processing, but also needs a consideration regarding poor reproducibility and secondary contamination of the amplified nucleic acid as a result.

In contrast, as a method for amplifying and measuring RNA conveniently, there is the method by Ishiguro et al. (see, Japanese Unexamined Patent Application Publication No. 2000-14400 and Ishiguro, T. et al., (2003) Analytical Biochemistry, 314, 77-86). This method measures changes in fluorescent characteristics during performing the RNA amplification method under the presence of a nucleic acid probe which is designed to change the fluorescent characteristics by intercalating a moiety of intercalating fluorescent dye into a region of a complementary double-stranded chain, when the probe is a nucleic acid probe labeled with intercalating fluorescent dye and forms a complementary double-strand chain with a target nucleic acid. The RNA amplification and the measurement can be conducted simultaneously, conveniently and quickly at a constant temperature, in one step, and in a tube.

**SUMMARY OF THE INVENTION**

### Problem to Be Solved by the Invention

As a target for nucleic acid amplification to detect about 50 *Legionella* species but not detect other *non-Legionella* bacteria, the target nucleic acid (target gene) that is highly conserved among *Legionella* species but significantly different from non-*Legionella* bacteria is considered desirable.

As one of the candidates of such nucleic acid target (target gene), 16S ribosomal RNA is considered. However, even if 16S ribosomal RNA is used as a target, there is greater diversity among *Legionella* species, and many of the sequences also show high homology with those of non-*Legionella* bacteria. So, it was extremely difficult to construct a primer set that amplified 16S ribosomal RNA of about 50 *Legionella* species with high efficiency and uniformly but would not amplify 16S ribosomal RNA from non-*Legionella* bacteria.

The present invention was made in view of the foregoing circumstances. It is an object of the present invention to provide a primer set for amplifying ribosomal RNA of a bacterium belonging to the genus *Legionella* specifically and highly efficiently, and a method for detecting ribosomal RNA of a bacterium belonging to the genus *Legionella* by using the primer set.

### Means for Solving the Problem

As a result of intensive studies to solve the forgoing problem, the inventors constructed a primer set for specifically amplifying ribosomal RNA of a bacterium belonging to the genus *Legionella* and a detection method using the primer set.

In other words, a first aspect in accordance with the invention provides a primer set capable of amplifying ribosomal RNA of a bacterium belonging to the genus *Legionella* as a target, comprising an antisense primer containing nucleotides of at least 15 or more bases hybridizing specifically with a nucleic acid of a base sequence of SEQ ID NO: 1 or 7 under stringent conditions, and a sense primer containing nucleotides of at least 15 or more bases hybridizing specifically with a nucleic acid of a base sequence of SEQ ID NO: 47 or 49 under stringent conditions.

A second aspect in accordance with the invention also provides a primer set capable of amplifying ribosomal RNA of a bacterium belonging to the genus *Legionella* as a target, comprising an antisense primer containing nucleotides of at least 15 or more bases having 80% or more homology with a portion of a base sequence of SEQ ID NO: 45 or 48, and a sense primer containing nucleotides of at least 15 or more bases having 80% or more homology with a portion of a base sequence of SEQ ID NO: 2 or 8.

Performing a variety of nucleic acid amplification methods by using these specific primer sets for ribosomal RNA of a bacterium belonging to the genus *Legionella* makes it possible to amplify ribosomal RNA of a bacterium belonging to the genus *Legionella* specifically and highly efficiently, and to detect a bacterium belonging to the genus *Legionella* conveniently and reliably.

An aspect in accordance with the invention also provides a method for detecting ribosomal RNA of a bacterium belonging to the genus *Legionella,* comprising the steps of:
obtaining amplified products by performing a nucleic acid amplification using the primer set and ribosomal RNA of a bacterium belonging to the genus *Legionella* as a template, and simultaneously or subsequently to the previous step; and
detecting the amplified products using a nucleic acid probe containing nucleotides of at least 15 or more bases having 80% or more homology with either a base sequence of SEQ ID NO: 6 or 11 or a complementary base sequence thereof labeled by means for generating a detectable signal.

This detection method makes it possible to conduct the amplification and detection of ribosomal RNA of a bacterium belonging to the genus *Legionella* simultaneously, and quickly and conveniently in a tube by performing a combination of the nucleic acid amplification method using the specific primer set of ribosomal RNA of a bacterium belonging to the genus *Legionella* and the detection using the specific labeled nucleic acid probe for ribosomal RNA of a bacterium belonging to the genus *Legionella.*

An aspect in accordance with the invention also provides a detection kit of ribosomal RNA of a bacterium belonging to the genus *Legionella* comprising the primer set in order to obtain amplified products by performing a nucleic acid amplification using ribosomal RNA of a bacterium belonging to the genus *Legionella* as a template; and
a nucleic acid probe containing nucleotides of at least 15 bases having 80% or more homology with either a base sequence of SEQ ID NO: 6 or 11 or a complementary base sequence thereof labeled by means for generating a detectable signal to detect the amplified products.

Using this detection kit makes it possible to perform a combination of the nucleic acid amplification method using the specific primer set for ribosomal RNA of a bacterium belonging to the genus *Legionella* and the detection using the specific labeled nucleic acid probe for ribosomal RNA of a bacterium belonging to the genus *Legionella,* and to perform the amplification and detection of ribosomal RNA of a bacterium belonging to the genus *Legionella* simultaneously, and quickly and conveniently in a tube.

### Effects of the Invention

Aspects in accordance with the present invention make it possible to detect ribosomal RNA of a bacterium belonging to the genus *Legionella* quickly with high sensitivity. Furthermore, the aspects, in comparison to conventional methods, improve specificity for detecting viable cells. Accordingly, the aspects also make it possible to reduce false positives in patients after treatment, environmental water after cleaning and the like.

### BRIEF DESCRIPTION OF THE DRAWINGS

Figure 1 shows a structure of an intercalating fluorescent dye-labeled nucleic acid probe produced in Example 2. B1, B2, B3, and B4 independently represent a base. A probe incorporating fluorescent intercalating dye (oxazole yellow) via a linker to the phosphodiester moiety according to the method by Ishiguro et al. (Ishiguro, T.et al, (1996) Nucleic Acids Res., 24, 4992-4997) is shown. Besides, to prevent elongation reaction from the 3' end-OH, the 3' end-OH has been modified by glycolic acid.
Figure 2a is a diagram to illustrate a representative base sequence of the invention.

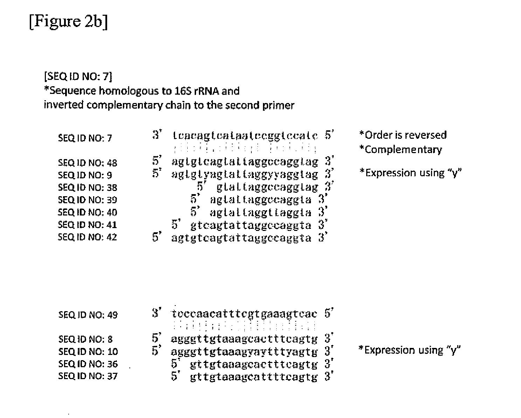

Figure 2b is a diagram to illustrate a representative base sequence of the invention.

### BEST MODE FOR CARRYING OUT THE INVENTION

### Description of the Terms

The term "a bacterium belonging to the genus *Legionella"* in the embodiments refers to a Gram-negative bacillus that is widely distributed in soil, fresh water, and the like in nature. A bacterium belonging to the genus *Legionella* is classified into about 50 species, and has the potential to develop legionellosis by all the species. A bacterium belonging to the genus *Legionella* in the embodiments includes not only the bacterium belonging to the genus *Legionella* by biological classification (genetic and molecular biological classification in particular) at the time of the application but also includes a bacterium belonging to the related genus *Legionella* (old *Legionella)* which is classified as other species at present. Such a bacterium belonging to the related genus *Legionella* includes, for example, *Fluoribacter, Tatlockia,* and the like. These bacteria are still important bacteria as disease causing bacteria (a bacterium belonging to the genus *Legionella*) for legionellosis. An aspect of the present invention can also detect these bacteria as the subject, thus, these bacteria are included as "a bacterium belonging to the genus *Legionella"* in the embodiments.

The term "ribosomal RNA" in the embodiments refers to each subunit of ribosomal RNA (rRNA). While ribosomal RNA includes subunits of ribosomal RNA differed in a sedimentation coefficient (S), a primer set of the invention targets primarily RNA constituting 16S subunits.

The terms "nucleotide" or "nucleic acid" in the embodiments refer to naturally-occurring bases, nucleotides or nucleosides containing sugar and an inter-sugar bond (including both RNA and DNA) and refer to a general term including an oligomer thereof (oligonucleotide, for example, about 2-100 bases) and a polymer thereof (polynucleotide, for example, more than 100 bases). The nucleotide or nucleic acid related to the embodiments includes a nonnative monomer which functions similarly, a monomer labeled with fluorescent molecules, etc., and radioactive isotopes, or an oligomer or polymer containing these.
The base symbol "y" represented in the sequence listing (SEQ ID NO: 3, 9, 10, 50) also represents cytosine (c) or thymine (t).

The term "primer" in the embodiments refers to nucleotides which hybridize with a template and is required for initiating a nucleic acid amplification reaction. Based on the desired template of the amplification in a nucleic acid amplification reaction, the primer can be designed to hybridize with its template and perform a nucleic acid amplification reaction, for example, to be capable of producing products (in a chain length or sequence) from a specific PCR method, LAMP method, ICAN method, NASBA method, TMA method, 3SR method, TRC method, and the like, preferably to include the template specific sequences by the primer itself. A typical primer can be designed to have a chain length of, but not limited to, usually 15 to 100 bases, preferably 15 to 35 bases.

Although the 3' region of a primer is preferably complementary to the template strand, a functional sequence such as recognition sequences of restriction enzymes, tags, etc., can also be added to the 5' region. As discussed below, when ICAN method, etc. as a nucleic acid amplification method is used, for example, the primer may also contain RNA instead of only DNA, if appropriate, or consist of only RNA. A suitable primer for the invention in particular is described below. In a PCR method or other concerted nucleic acid amplification methods, in general, a primer set including a pair of a sense primer (the first primer) and antisense primer (the second primer) or including more primers can be used.

The term "specifically hybridize" in the embodiments refers to condition, wherein certain nucleotides bind complementarily to other nucleotides through a hydrogen bond, etc., and not to the control nucleotides under the same conditions. The nucleotides do not necessarily have specificity to all the other nucleotides, these only need to have the specificity depending on the intended use. For example, it can also be referred to "specifically hybridize" if the nucleotides do not bind to the nucleotides from other than ribosomal RNA of a bacterium belonging to the genus *Legionella* in the detection.

The hybridization conditions can be selected depending on the intended use of the nucleotides. For example, if the nucleotides are used as a primer for PCR method, these are selected to hybridize at annealing conditions in PCR method. When used as a nucleic acid probe, these are selected to hybridize under the hybridization conditions. Preferably, these are selected to hybridize under the stringent hybridization conditions.

The "stringent conditions" in the embodiments also include, but are not limited to, the condition containing a denaturation agent such as formamide, etc., during the hybridization, for example, the condition at 42°C having 50% (v / v) formamide and 0.1% bovine serum albumin / 0.1°/ ficoll / 0.1% polyvinylpyrrolidone / 50 mM sodium phosphate buffer at pH 6.5, 750 mM NaCl, and 75 mM sodium citrate, and the modified condition thereof. Hybridization temperature conditions, which can be determined depending on the nucleic acid amplification reaction or hybridization reaction in use, can also be, for example, 42°C, 43°C, 45°C, 50°C, 55°C, 60°C, 65°C, 68°C, 70°C, or 72°C.

Any methods can be used if the "nucleic acid amplification" methods are known in the art as a method for amplifying the target nucleic acid in the embodiments. But the methods using a primer and a primer set such as, for example, PCR method, LAMP method or other concerted nucleic acid amplification methods can also be used. When RNA is amplified in particular, RT-PCR method (a kinetic RT-PCR method and the like in which cDNA is synthesized by reverse transcriptase using RNA as a template, simultaneously or subsequently to this step, and the nucleic acid product derived from the target RNA is amplified by heat-resistant DNA polymerase), RT-LAMP method or other concerted nucleic acid amplification methods can be used.

Furthermore, as an RNA amplification method, in addition to the foregoing LAMP method, NASBA method (see, Japanese Patent No. 2,650,159 and No. 3,152,927), TMA method (see, Japanese Patent No. 3,241,717), 3SR method and the like can also be used as a method which amplifies only RNA at a constant temperature. These methods in the overview perform a chain reaction comprising synthesizing double-stranded DNA that contains a promoter sequence by a primer containing a promoter sequence for the target RNA, reverse transcriptase, if appropriate, and ribonuclease H (RNaseH); producing RNA containing a specific nucleic acid sequence derived from the target RNA by RNA polymerase using the double-stranded DNA as a template, and subsequently; becoming the RNA template for a synthesis of the double-stranded DNA containing the promoter sequence.

Furthermore, as other nucleic acid amplification methods, ICAN method (for example, see Japanese Patent No. 3,433,929) can also be used. These methods in the overview perform a strand displacement chain reaction, comprising synthesizing cDNA using RNA as a template by reverse transcriptase for the target RNA, simultaneously or subsequent to this; synthesizing the double-stranded DNA containing RNA by a chimeric primer containing RNA-DNA and transcriptase; nicking the double-stranded DNA by ribonuclease H (RNaseH); and producing DNA containing a specific nucleic acid sequence derived from the target RNA.

For a detection method of the amplified products in the embodiments, any methods, if known in the art as a method for detecting a nucleic acid, can also be used. For example, a detection of the amplified nucleic acid by gel electrophoresis, or a detection by a hybridization method using a nucleic acid probe linked by a detectable label may be used. In addition, following methods, which have advantages in processing of multiple samples, automation, reproducibility, secondary contamination, and the like, may also be used.

For a simple method for detecting RNA, but is not limited to, a nucleic acid probe labeled with intercalating fluorescent dye can be used (see the part describing the probe in Japanese Unexamined Patent Application Publication No. 2000-14400 and Ishiguro, T. et al., (2003) Analytical Biochemistry, 314, 77-86). The detection method using such a probe measures changes in fluorescent characteristics during performing a nucleic acid amplification method under the presence of a nucleic acid probe which is designed to change the fluorescent characteristics by intercalating a moiety of intercalating fluorescent dye into a region of a complementary double-stranded chain when the probe forms a complementary double-strand chain with a target nucleic acid. By using this method, the nucleic acid amplification and detection (measurement) can also be conducted simultaneously, conveniently and quickly at a constant temperature, in one step, and in a tube.

The methods for detecting the amplified nucleic acid are not limited to the above. For example, LAMP method may detect cloudiness from a by-product magnesium pyrophosphate as an indicator. When the methods are known detection methods of the amplified product that are applicable to each nucleic acid amplification method, any methods can also be used even if the methods do not directly measure the amplified nucleic acid.

Furthermore, for methods easily performing RNA amplification and a detection thereof, for example, TRC (Transcription Reverse-transcription Concerted reaction) method (described in Japanese Unexamined Patent Application Publication No. 2000-14400 and Ishiguro, T. et al., (2003) Analytical Biochemistry, 314, 77-86) can also be used. TRC method is a real-time detection method of RNA amplification, which combines TRC reaction (wherein the reverse transcription reaction and transcription reaction proceed concertedly) and INAF (Intercalation Activating Fluorescence probe) probe having a structure in which intercalating fluorescent dye binds to the phosphorus atom of phosphodiester of an oligonucleotide chain via a linker. In the TRC reaction, for example, the reaction forms a cycle between reverse transcription reaction and transcription reaction(in which reverse transcriptase and RNA polymerase act concertedly at a constant temperature of 43°C); and an exponential amplification of RNA is achieved promptly. Since INAF probe exhibits fluorescence intensification when the probe binds complementarily to the amplified RNA in a specific manner, the amplification of RNA can be monitored in real time if the probe co-exists from the early stages of the TRC reaction.
These methods can be practiced according to the instructions included in the kit or conventional protocols.

The term "promoter sequence of RNA polymerase" in the embodiments also refers to a promoter sequence required for RNA polymerase to initiate transcription. For example, when various nucleic acid amplification methods such as NASBA method, TMA method, 3 SR method, and the like are performed, the primers are required to have a promoter sequence of RNA polymerase. For such a promoter sequence of RNA polymerase, any promoter sequences may also be used if the promoter sequence can be recognized by RNA polymerase that is used for each nucleic acid amplification method. Specific examples of the promoter sequence of RNA polymerase include the promoter sequence of, for example, T7 RNA polymerase, SP6 RNA polymerase or T3 RNA polymerase. The promoter sequence of RNA polymerase is added, preferably, to the 5' end of a primer.

The term "labeled by means for generating a detectable signal" in the embodiments also refers to being labeled by means for generating any signals that can be used in the field of biology and medicine. The means include, for example, a radioactive label by α-³³P and the like, a fluorochrome-label by a fluorescent intercalator and the like, dye-mark by color compounds and the like, or an enzyme-label by HRP, AP, and the like. Preferred is a fluorochrome-label. The labels are also, more preferably, those which change characteristics of the label by forming a complementary double-strand, furthermore preferably, an intercalating fluorochrome-label which changes the fluorescence properties by forming a complementary double-strand. Those skilled in the art can easily perform these labeling according to the instructions using various labeling reagent kits or conventional methods.

The term "homology" in the embodiments is defined as a percentage of the number of the same nucleic acid bases in a candidate sequence to the intended bases, in which the sequences are aligned and gaps are inserted, if appropriate, to achieve the maximum percent sequence identity. On this occasion, DNA and RNA having the same bases (thymine and uracil are also considered as identical) are defined as having the identical nucleic acid bases because they have very similar binding characteristics. Although the alignment in order to determine the homology can be achieved by using various methods in the technical scope of those skilled in the art such as publicly available computer software including, for example, blast, blast2seq, or ALIGN provided by NCBI (National Center for Biotechnology Information), the value calculated by using the initial standard parameters in blast2seq can also be used.

Besides, in terms of the primers for nucleic acid amplification, other probes, and the like, the base sequences which have more than 80% , 85%, 90%, 95%, 98% or more (100% or less) of homology with the base sequences instead of a certain base sequence or the base sequences having a substitution, deletion, and insertion of 1 base, 2 bases, 3 bases, 4 bases, 5 bases or several bases for the certain base sequence, can also be used (in a way similar to the definition of homology, the substitution between RNA and DNA having the same bases is not included in a substitution, deletion, and insertion herein).

The term "sufficiently complementary sequence" in the embodiments refers to the sequence, which can highly efficiently hybridize with ribosomal RNA of a bacterium belonging to the genus *Legionella* and the perfectly homologous nucleic acid thereof under nucleic acid amplification reaction conditions (salt concentrations, reagent compositions such as oligonucleotide concentrations, etc., and temperatures). The term "sufficiently homologous sequence" in the embodiments also refers to the sequence, which can highly efficiently hybridize with the perfectly complementary sequence to ribosomal RNA of a bacterium belonging to the genus *Legionella* under nucleic acid amplification reaction conditions. In other words, the terms "sufficiently complementary sequence" or "sufficiently homologous sequence" in the embodiments are not necessarily to be a perfectly complementary or perfectly homologous sequence respectively within the range not affecting the hybridization efficiency in the nucleic acid amplification reaction.

### Embodiments

The embodiments of the invention are described below.

The certain embodiments of the invention are a primer set capable of amplifying ribosomal RNA of a bacterium belonging to the genus *Legionella* as a target, comprising an antisense primer containing at least 15 or more bases hybridizing specifically with a nucleic acid of a base sequence of SEQ ID NO: 1 (actgatacag gtgctgca) or 7 (ctacctggcc taatactgac act) under stringent conditions, and a sense primer containing nucleotides of at least 15 or more bases hybridizing specifically with a nucleic acid of a base sequence of SEQ ID NO: 47(tataaccaac agctagttga catcgtttac agcgtgg) or 49 (cactgaaagt gctttacaac cct) under stringent conditions.

The certain embodiments of the invention are also a primer set capable of amplifying ribosomal RNA of a bacterium belonging to the genus *Legionella* as a target, comprising an antisense primer containing nucleotides of at least 15 or more bases having 80% or more homology with a portion of a base sequence of SEQ ID NO: 45 (tgcagcacct gtatcagt) or 48 (agtgtcagta ttaggccagg tag), and a sense primer containing nucleotides of at least 15 or more bases having 80% or more homology with a portion of a base sequence of SEQ ID NO: 2 (ccacgctgta aacgatgtca actagctgtt ggttata) or 8 (agggttgtaa agcactttca gtg).

The foregoing embodiments may be a primer set for amplifying ribosomal RNA of a bacterium belonging to the genus *Legionella* as a target, comprising an antisense primer containing at least 15 nucleotides having a sufficiently complementary sequence to SEQ ID NO: 1, and a sense primer containing at least 15 nucleotides having a sufficiently homologous sequence to SEQ ID NO: 2.

The foregoing embodiments may be a primer set for amplifying ribosomal RNA of a bacterium belonging to the genus *Legionella* as a target, comprising an antisense primer containing at least 15 nucleotides having a sufficiently complementary sequence to SEQ ID NO: 7, and a sense primer containing at least 15 nucleotides having a sufficiently homologous sequence to SEQ ID NO: 8.

In other words, the primers included in the foregoing primer sets have the homologous or complementary base sequences to a portion of a ribosome RNA sequence of a bacterium belonging to the genus *Legionella* (i.e., having primer binding sites on SEQ ID NO: 1 and 47, or SEQ ID NO: 7 and 49), preferably, for example, specifically hybridize with a nucleic acid of a base sequence of SEQ ID NO: 1, 47, 7, 49 under stringent conditions, or have 80% or more homology with a portion of a base sequence of SEQ ID NO: 45, 2, 48, 8.

Amplifying nucleic acid by using the foregoing primer set makes it possible to detect a bacterium belonging to the genus *Legionella,* specifically and quickly with high sensitivity. Furthermore, the specificity to viable cells is also improved more than that of conventional methods. Thus, the embodiments of the invention also make it possible to reduce false positives in patients after treatment, environmental water after cleaning, and the like.

By performing nucleic acid amplification of ribosomal RNA of a bacterium belonging to the genus *Legionella* as a target using the primer set, the detection of a bacterium belonging to the genus *Legionella* also becomes much shorter to take 10 minutes to several hours. The detection by culture methods takes 5 to 7 days.
A conventional immunological test using antibodies specific for *Legionella pneumophila* (the test can be commonly used for the detection of a bacterium belonging to the genus *Legionella*) is limited to *Legionella pneumophila* and has insufficient sensitivity to other species. However, the methods using this primer sets can detect a broad range of bacteria belonging to the genus *Legionella.*

In the detection of *Legionella* DNA as a target using as a conventional molecular biological approach, there are possibilities to detect false positives in patients after treatment, environmental water after cleaning, and the like because the target DNA is relatively stable in dead bacteria. In contrast, the embodiments of the present invention make it possible to examine viable cells specifically by targeting RNA that is susceptible to degradation in dead bacteria (see, Steve. A. et al., (2006) Antimicrobial agents and chemotherapy, 50, 1913-1920). Since the amount of 16S ribosomal RNA contained in a single bacterium is 1000 to 10000 times in comparison to 16S ribosomal DNA, more sensitive detections can particularly be conducted.

Even if 16S ribosomal RNA (and/or DNA) is targeted, in reality, the RNA (and/or DNA) has a rich diversity in species among bacteria belonging to the genus *Legionella,* and many show high homology with non-*Legionella* bacteria. However, a more preferred primer set of the invention has specificity for amplifying about 50 species of 16S ribosomal RNA of a bacterium belonging to the genus *Legionella* highly efficiently and uniformly, and not amplifying 16S ribosomal RNA of non-*Legionella* bacteria.

Here, the homology between the above primers and the base sequences of SEQ ID: 45, 2, 48, 8 is preferably more than 85%, 90%, 95%, 98% or more (100% or less). Alternatively, the primers including the base sequences which have a substitution, deletion, and insertion of 1 base, 2 bases, 3 bases, 4 bases, 5 bases or several bases to the above base sequences, can also be used (in a way similar to the definition of homology, the substitution between RNA and DNA having the same bases is not included in a substitution, deletion, and insertion herein). The continuous sequence (or the substituted sequence thereof) within the above base sequences is furthermore preferred.

To each primer, depending on its purpose, may even be added various things. Considering the effectiveness as a primer, its length is at least more than 15 bases, 18 bases, or 20 bases. The maximum length is not particularly limited. But considering the low efficiency of the nucleic acid amplification reaction with primers that are too long, or considering the length of primers required for each amplification reaction, the upper limit can be less than 100 bases, 80 bases, 60 bases or 50 bases.

The nucleic acid amplification methods using RNA as a target include RT-PCR method, RT-LAMP method, NASBA method, TMA method, 3SR method, ICAN method, TRC method and the like. Any of the foregoing methods can use a primer set of the invention.

The further embodiments of the invention are also a primer set capable of amplifying ribosomal RNA of a bacterium belonging to the genus *Legionella* as a target, comprising an antisense primer containing nucleotides of at least 15 or more bases within a base sequence of SEQ ID NO: 3 (tgyagyayyt gtatyagt) or 9 (agtgtyagta ttaggyyagg tag), and the sense primer. The base sequence of SEQ ID NO: 3 or 9 indicates the base sequence in which a part of cytosine within the sequence of SEQ ID NO: 45 or 48 is substituted by thymine.

The foregoing embodiments may be a primer set, comprising an antisense primer containing at least 15 nucleotides of the sequence shown in SEQ ID NO: 3, wherein the above antisense primer has a sufficiently complementary sequence to SEQ ID NO: 1 and contains bases of cytosine substituted by thymine.

The foregoing embodiments may also be a primer set, comprising an antisense primer containing at least 15 nucleotides of the sequence shown in SEQ ID NO: 9, wherein the above antisense primer has a sufficiently complementary sequence to SEQ ID NO: 7 and contains bases of cytosine substituted by thymine.

The further embodiments of the invention are also a primer set capable of amplifying ribosomal RNA of a bacterium belonging to the genus *Legionella* as a target, comprising the foregoing sense primer and a sense primer containing nucleotides of at least 15 or more bases within a base sequence of SEQ ID NO: 50 (yyaygytgta aaygatgtya aytagytgtt ggttata) or 10 (agggttgtaa agyaytttya gtg). The base sequences shown in SEQ ID NO: 50 or 10 indicate the base sequences in which a part of cytosine within the sequence of SEQ ID NO: 2 or 8 is substituted by thymine.

The foregoing embodiments may be a primer set, comprising a sense primer containing a sequence shown in SEQ ID NO: 50, wherein the above sense primer containing at least 15 nucleotides has a sufficiently homologous sequence to SEQ ID NO: 2 and contains bases of cytosine substituted by thymine.

The foregoing embodiments may also be a primer set, comprising a sense primer containing at least 15 nucleotides of a sequence shown in SEQ ID NO: 10, wherein the above sense primer has a sufficiently homologous sequence to SEQ ID NO: 8 and contains bases of cytosine substituted by thymine.

In order to demonstrate the high specificity in nucleic acid amplification, the desired is a system that is significantly less efficient than those without a mismatch when there is even one mismatch between the target RNA and the primer. In other words, in the present invention, the sequence in which at least a part of cytosine within the primer is substituted by thymine can be used as a preferred embodiment to improve the specificity for a bacterium belonging to the genus *Legionella.*

It has been reported that guanine (G) in a target RNA and thymine (dT) in a primer can form relatively stable base pairs (see, Naoki. S. et al., (2000) Biochemistry, 39, 11270-11281). So, when the primer does not have a mismatch originally with the target RNA (for specific annealing), the hybridization efficiency is not reduced significantly even if the appropriate numbers of cytosine in the sequence are substituted by thymine. However, when there is even one mismatch with the target RNA (for nonspecific annealing), it is considered that the hybridization efficiency is reduced significantly by introducing a substitution from cytosine to thymine in the sequence other than the mismatch bases thereof.

Thus, by using the primer set, such as, for example, the foregoing primer set, for which cytosine in the primer sequence is substituted by thymine, the method makes it possible to reduce the hybridization efficiency for ribosomal RNA of non*-Legionellα* bacteria significantly while maintaining the hybridization efficiency for ribosomal RNA of a bacterium belonging to the genus *Legionella* with this primer set.

The further embodiments of the invention are a primer set comprising an antisense primer containing nucleotides of a base sequence in which the antisense primer is shown in SEQ ID NO: 4 (tagcatctgt atcagt). The base sequence shown in SEQ ID NO: 4 is a particularly effective sequence among the base sequences shown in SEQ ID NO: 3.

The foregoing embodiments may be a primer set, wherein the above antisense primer containing at least 15 nucleotides of the sequence shown in SEQ ID NO: 3 comprises the sequence shown in SEQ ID NO: 4.

By using these primer sets having the specific sequence as a primer sequence, more reliable and specific nucleic acid amplification and the detection of a bacterium belonging to the genus *Legionella* can be performed.

The certain embodiments of the present invention are the primer sets, wherein at least one of antisense and sense primers further comprises a sequence which plays a role in the specific functions such as a promoter sequence of RNA polymerase, a sequence constituting a stem-loop structure, a restriction enzyme site and the like.

As the primer contains a promoter sequence, the nucleic acid amplification methods such as NASBA method, TMA method, 3SR method, and the like can be used. Conventional common PCR methods require rapid change of the reaction temperature. This is a barrier for labor-saving and low-cost of reaction devices in automation. In contrast, the foregoing nucleic acid amplification methods have such advantages, wherein the amplification reaction is isothermal, proceeds continuously, has high amplification efficiency, requires no special equipment, and can amplify in one step in a fashion similar to DNA even if the template is even RNA. The methods are extremely effective when used in the diagnosis.

If a promoter sequence is added to a sense primer, the target RNA requires to be cleaved at the 5' end of the specific nucleic acid sequence. The specific nucleic acid sequence refers to the base sequence that is from the 5' end of the homologous region to the sense primer on the target RNA to the 3' end of the complementary region to the antisense primer. For the method cleaving the target RNA in such a manner, the method, wherein oligonucleotide DNA (oligonucleotide for cleavage) having the complementary sequence to a region adjacent to a portion of 5' end overlap within the specific nucleic acid sequence in the 5' direction is added, and the RNA portion of the RNA-DNA hybrid, formed as a result, is cleaved by ribonuclease H (RNaseH) activity, is preferred. As the oligonucleotide for cleavage needs to prevent a elongation reaction from the 3' end, those which have been chemically modified at the 3' end hydroxyl group such as, for example, modified by amination and the like, are desirable to be used.

The nucleic acid amplification using the primer set of the invention can be performed by a nucleic acid amplification method, comprising synthesizing cDNA by the foregoing antisense primer using ribosomal RNA of a bacterium belonging to the genus *Legionella* as a template, either simultaneously or subsequently to this step; and performing a chain reaction by the foregoing primer set and appropriate DNA polymerase activity and/or RNA polymerase activity.

Amplified products obtained using the foregoing nucleic acid amplification methods can be detected by publicly known nucleic acid detection methods. Furthermore, the amplified products can also be detected specifically by a nucleic acid probe labeled by means for generating a detectable signal.

The sequence which plays a role in a variety of specific functions, such as stem-loop structures, restriction enzyme sites, and the like can also be added to and included in these primers. In respect to the sequences responsible for these specific functions, any functional sequences used in the field of genetic engineering, particularly a variety of sequences responsible for the specific functions used in nucleic acid processing, amplification, and detection, can be used. They can be designed in accordance with the methods normally used in such areas.

The certain embodiments of the invention are a method for detecting ribosomal RNA of a bacterium belonging to the genus *Legionella,* comprising the steps of: obtaining amplified products by performing a nucleic acid amplification using the primer set and ribosomal RNA of a bacterium belonging to the genus *Legionella* as a template, simultaneously or subsequently to the previous step; and
detecting the amplified products by a nucleic acid probe containing nucleotides of at least 15 bases having 80% or more homology with either a base sequence shown in SEQ ID NO: 6 (cactgtatgt caagggtagg t) or 11 (acctacgcac cctttacg) or a complementary base sequence thereof labeled by means for generating a detectable signal.

This detection method makes it possible to perform the amplification and detection of ribosomal RNA of a bacterium belonging to the genus *Legionella,* without a separation analysis, at a constant temperature, in one step, simultaneously, and quickly and conveniently in a tube by performing a combination of the nucleic acid amplification method using the specific primer set for the ribosomal RNA and the detection using the specific labeled nucleic acid probe for the ribosomal RNA.

The above detection method can also be a method for detecting ribosomal RNA of a bacterium belonging to the genus *Legionella,* wherein the step amplifying nucleic acid and obtaining the amplified products comprises the steps of:
(1) producing double-stranded DNA comprising a promoter sequence and a specific base sequence downstream of the promoter sequence by a first primer homologous to at least a portion of a downstream region from a 5' end of a specific base sequence of ribosomal RNA of a bacterium belonging to the genus *Legionella* and a second primer complementary to at least a portion of a upstream region from a 3' end of a specific base sequence (at least one of the first and second primers has a promoter sequence at its 5' end);
(2) producing RNA transcripts using the double-stranded DNA as a template;
(3) amplifying the RNA transcripts in a chain reaction in which the RNA transcripts continuously become a template for DNA synthesis.

The certain embodiments of the invention are a detection kit of ribosomal RNA of a bacterium belonging to the genus *Legionella* comprising the primer set in order to obtain amplified products by performing a nucleic acid amplification using ribosomal RNA of a bacterium belonging to the genus *Legionella* as a template; and
a nucleic acid probe containing at least 15 nucleotides having 80% or more homology with either a base sequence shown in SEQ ID NO: 6 or 11 or a complementary base sequence thereof labeled by means for generating a detectable signal to detect the amplified products.

Using the detection kit makes it possible to perform a combination of the nucleic acid amplification method using the specific primer set for ribosomal RNA of a bacterium belonging to the genus *Legionella* and the detection using the specific labeled nucleic acid probe for the ribosomal RNA. This makes it possible to perform the amplification and detection of ribosomal RNA of the bacterium belonging to the genus *Legionella* at a constant temperature, in one step, simultaneously, and conveniently and quickly in a tube.

The above detection kit can also be a detection kit, further comprising at least RNA-dependent DNA polymerase, ribonuclease H (RNaseH), DNA-dependent DNA polymerase, and RNA polymerase.

The intercalating fluorescent dye-labeled oligonucleotide sequence used in the embodiments can be those capable of hybridizing with at least a portion of the sequence in the nucleic acid amplification products, preferably be nucleotides of at least 15 bases having 80% or more homology with either a base sequence shown in SEQ ID NO: 6 or 11 or a complementary base sequence thereof. More preferably, the homology is more than 85%, 90%, 95%, 98% or more (100% or less). Alternatively, the nucleotides including the base sequence which has a substitution, deletion, and insertion of 1 base, 2 bases, 3 bases, 4 bases, 5 bases or several bases to the above base sequences, can also be used. Furthermore preferably, the nucleotides are a continuous sequence. In addition, to inhibit a elongation reaction from the 3' end of the oligonucleotide, a hydroxyl group of the 3' end is preferably chemically modified (for example, addition of glycolic acid).

Using RT-LAMP method, NASBA method, TMA method, 3SR method, and the like as a nucleic acid amplification method has the additional advantages compared to RT-PCR method that is conventionally used. RT-PCR method requires a two-step process of the reverse transcriptase (RT) and PCR processes. This can be not only an aggravating factor in the reproducibility due to complicated operations, but also increase a risk of secondary contamination. In addition, these processes usually take longer than two hours, and there is a problem in respect to the processing of multiple samples and the reduction of testing costs.

A kinetic RT-PCR method, which performs PCR process under the presence of intercalating fluorescent dye to measure the increase in fluorescence over time, is also widely used. The method can conduct PCR process and the measurement process at least 20 minutes. Unfortunately, the method also detects nonspecific amplification products such as a primer dimer and the like. In addition, PCR method requires rapid change of the reaction temperature. This is a barrier for labor-saving and low-cost of reaction devices in automation. Since RT-PCR method can also amplify DNA, DNase treatment and the like of samples to completely remove DNA are required when the target is RNA as described above. The operation can be more complicated due to this process.

For these problems, using the methods such as, for example, RT-LAMP method, NASBA method, TMA method, 3SR method, ICAN method, and the like, makes it possible to perform the measurement in a more convenient one-step process and in a short time, and is advantageous in respect to the processing of multiple samples and the reduction of testing costs.

As more preferred embodiments, TRC method (see, Japanese Unexamined Patent Application Publication No. 2000-14400 and Ishiguro, T. et al., (2003) Analytical Biochemistry, 314, 77-86) can be included. By using TRC method, one can perform from the amplification to detection at a constant temperature, in one step, and quickly and conveniently in a tube.

The certain embodiments of the invention are a method for detecting ribosomal RNA of a bacterium belonging to the genus *Legionella* by performing a nucleic acid amplification under the presence of a nucleic acid probe containing an intercalating fluorescence dye-labeled sequence shown in SEQ ID NO: 6 or a complementary sequence thereof, and by measuring changes of the fluorescence intensity of a reaction solution, the method comprising the steps of:
performing cDNA synthesis by an antisense primer containing a sequence shown in SEQ ID NO: 4 and by RNA-dependent DNA polymerase using ribosomal RNA of a bacterium belonging to the genus *Legionella* as a target;
degrading and dissociating a portion of RNA of the produced RNA-cDNA hybrid by RNaseH activity and synthesizing double-stranded DNA by a sense primer shown in SEQ ID NO: 5 and by DNA-dependent DNA polymerase using the cDNA as a template;
producing RNA transcripts by RNA polymerase using the double-stranded DNA as a template, in which one of the primer sets containing the antisense primer and the sense primer used herewith has a promoter sequence of RNA polymerase at its 5' end and the produced double-stranded DNA has a functional promoter at its 5' end; and
a nucleic acid amplification process in which the RNA transcripts become a template for the cDNA synthesis and each process is repeated in a chain reaction.

The certain embodiments of the invention are also a method for detecting ribosomal RNA of a bacterium belonging to the genus *Legionella* by performing a nucleic acid amplification under the presence of a nucleic acid probe containing an intercalating fluorescence dye-labeled sequence shown in SEQ ID NO: 6 and by measuring changes of the fluorescence intensity of a reaction solution in the processes, comprising the steps of:
cleaving ribosomal RNA at a 5' end of a specific nucleic acid sequence of ribosomal RNA of a bacterium belonging to the genus *Legionella* by oligonucleotide DNA containing a sequence shown in SEQ ID NO: 31 (catcgtttac agcgtgg) and by RNaseH activity using ribosomal RNA of a bacterium belonging to the genus *Legionella* as a target;
performing cDNA synthesis by an antisense primer containing a sequence shown in SEQ ID NO: 4 and by RNA-dependent DNA polymerase activity;
degrading and dissociating a portion of RNA of the produced RNA-cDNA hybrid by RNaseH activity and synthesizing double-stranded DNA by a sense primer shown in SEQ ID NO: 5 to which a promoter sequence is added to its 5' end and by DNA-dependent DNA polymerase using the cDNA as a template;
producing RNA transcripts by RNA polymerase using the double-stranded DNA as a template; and
a nucleic acid amplification process in which the RNA transcripts become a template for the cDNA synthesis and each process is repeated in a chain reaction.

In all the foregoing embodiments, the enzymes responsible for RNaseH activity, RNA-dependent DNA polymerase (reverse transcriptase) activity, and DNA-dependent DNA polymerase (transcriptase) activity are not particularly limited. However, reverse transcriptase that retains all of the activity is preferably used. The reverse transcriptase can use any known reverse transcriptase, that can be used in molecular biology experiments and the like. In particular, AMV reverse transcriptase, MMLV reverse transcriptase, HIV reverse transcriptase, etc., and a derivative thereof are preferably used.

Preferred RNA polymerase includes, but is not limited to, T7 RNA polymerase derived from bacteriophage, SP6 RNA polymerase, T3 RNA polymerase, etc., and a derivative thereof.

When this primer set in LAMP method and the like is used, the set can include 4 pairs of primer sets further containing primers for an external region of this sequence as a target. In this manner, according to the needs of each nucleic acid amplification method, more additional primers can be used. The number, the sequence, and the like of such additional primers can easily be designed in accordance with each nucleic acid amplification method and routine procedure.

The primer sets and detection methods described by the above embodiments do not limit the present invention and are disclosed for illustrative purposes. The technical scope of the present invention is determined by the description of the claims. Those skilled in the art can perform a variety of design changes within the technical scope of the invention described in the claims.

As a variety of design changes, for example, based on the embodiments, the base sequences suitable for a sense primer, instead of SEQ ID NO: 2 of the above embodiments, can use any one of the sequences including SEQ ID NO: 12 (ccacgctgta aacgatgtca a), 13 ( aaacgatgtc aattagctgt t), 14 (aaacgatgtc aactagctgt t), 15 (taaacgatgt caattagctg ttg), 16 (tgtaaacgat gtcaattagc tgttg), 17 (gctgtaaacg atgtcaatta gctgttg), 18 (gtcaactagc tgttggttat a), 51 (caactagctg ttggttatat ga), 52 (aaacgatgtc aactagctgt tggttatatg a), 53 (aaacgatgtc aactagctgt tggtta), or 54 (aaacgatgtc aactagctgt tggttata). The base sequences suitable for an antisense primer, instead of SEQ ID NO: 45 of the above embodiments, can use any one of the suitable sequences from SEQ ID NO: 19 (cagcacctgt atcagt), 20 (tagtatttgt attagt), 21 (tagtatttgt atcagt), 22 (tagtatctgt attagt), 23 (tagtacctgt attagt), 24 (tagcacttgt attagt), 25 (cagtacttgt attagt), 26 (tagtatctgt atcagt), 27 ( tagtacctgt atcagt), 28 (tagcacttgt atcagt), or 29 (cagtacttgt atcagt). In this case, as a base sequence for an intercalating fluorescent dye-labeled fluorescent probe, instead of SEQ ID NO: 6, SEQ ID NO: 44 (ctgtatgtca agggta) can also be used. As a base sequence for an oligonucleotide for cleavage, any one of the suitable sequences from SEQ ID NO: 30 (cgtggactac cagggtat), 31 (catcgtttac agcgtgg), 32 (gtttacagcg tggacta), 33 (ttacagcgtg gactacc), 34 (tacagcgtgg actacca), 35 (ttgacatcgt ttacagc ), or 55 (agctagttga catcgtttac ag) can be used.

Alternatively, as a base sequence suitable for a sense primer, instead of SEQ ID NO: 8 of the above embodiments, SEQ ID NO: 36 (gttgtaaagc actttcagtg) or 37 (gttgtaaagc attttcagtg) can be used. As a base sequence suitable for an antisense primer, instead of SEQ ID NO: 48 of the above embodiments, any one of the suitable sequences from SEQ ID NO: 38 (gtattaggcc aggtag), 39 (agtattaggc caggta), 40 (agtattaggt taggta), 41 (gtcagtatta ggccaggta), or 42 (agtgtcagta ttaggccagg ta) can also be used. As a base sequence for an oligonucleotide for cleavage, SEQ ID NO: 43 (acaaccctca ggcctt) can also be used.
These modified embodiments are included in the technical scope of the invention.

### EXAMPLES

Although the aspects of the invention are illustrated specifically by examples below, these are one example and the invention is not limited to those. The commercial reagents that are referred to in the examples, otherwise it was particularly shown, were used in accordance with the instructions by the manufacturer.

### Example 1

16S ribosomal RNA of *Legionella pneumophila* was prepared by performing *in vitro* transcription using the double-stranded DNA having 16S ribosomal DNA of *Legionella pneumophila* (including the base number 1-1397, the base number is in accordance with National Center Biotechnology Information accession No.AF122885) downstream of SP6 phage RNA polymerase promoter as a template, and subsequently by purifying RNA after complete digestion of the double-stranded DNA by DNasel treatment. This RNA was quantified by measuring the absorbance at 260 nm. In the following examples, this RNA was used as a standard sample of ribosomal RNA of a bacterium belonging to the genus *Legionella* that is a subject to be measured in the invention.

### Example 2

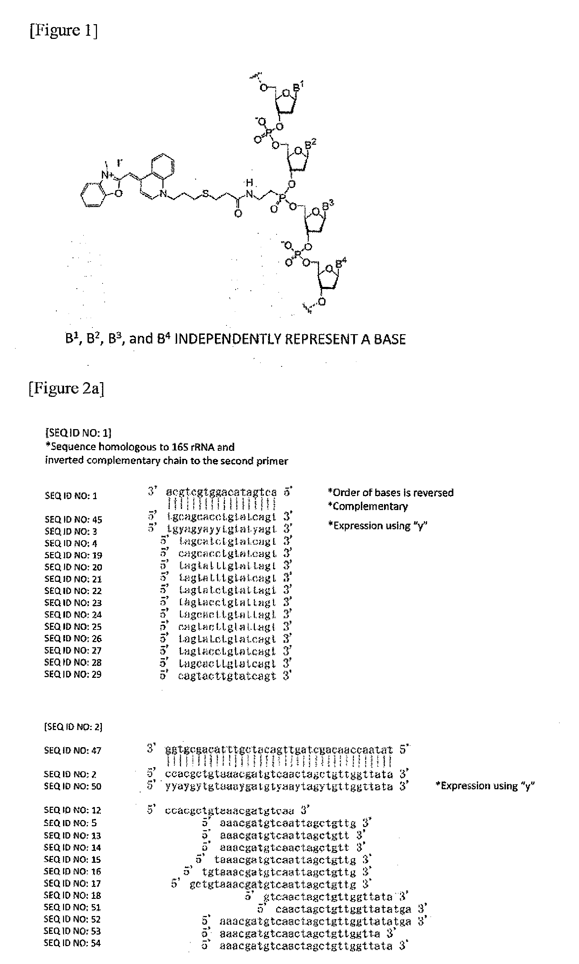

By following procedures, an oligonucleotide probe labeled with the intercalating fluorescent dye was prepared. First, by the method of Ishiguro et al. (Ishiguro, T. et al., (1996) Nucleic Acids Res., 24, 4992-4997), oxazole yellow-labeled nucleic acid probe in which oxazole yellow is linked via a linker to the phosphodiester moiety between the third C and fourth T from the 5' end of the sequence shown in SEQ ID NO: 6, between the eighth C and ninth A from the 5' end of the sequence shown in SEQ ID NO: 11, and between the ninth C and tenth A from the 5' end of the sequence shown in SEQ ID NO: 44, was prepared (Figure 1).

### Example 3

In the method of the invention, 16S ribosomal RNA of a bacterium belonging to the genus *Legionella* was measured using various combinations of the first primer, the second primer, the intercalating fluorescent dye-labeled nucleic acid probe, and the oligonucleotide for cleavage.

(1) The 16S ribosomal RNA of *Legionella pneumophila* (including the base number 1-1397) prepared in Example 1, was diluted to 10⁶, 10³, 10², or 50 copies/5 µl using the RNA diluent (10 mM Tris HCl (pH 8.0), 1 mM EDTA, 0.25 U/µl ribonuclease inhibitor, and 5.0 mM DTT), and was used as an RNA sample. (2) 20 µl of reaction solution of the following composition was aliquoted in 0.5 ml volume PCR tubes (Gene Amp Thin-Walled Reaction Tubes, manufactured by Perkin Elmer), and 5µl of the RNA sample was added thereto.

[The composition of reaction solution: concentration is the final concentration after addition of enzyme solution (in 30 µl)]
60 mM Tris· HCl(pH8.6);
18 mM magnesium chloride;
100 mM potassium chloride;
1 mM DTT;
Each 0.25 mM dATP, dCTP, dGTP, dTTP;
Each 3 mM ATP, CTP, UTP, GTP;
3.6 mM ITP;
1 µM of the first primer (SEQ ID NO is listed in Table 1): T7 polymerase promoter sequence (SEQ ID NO: 46) was added to the 5' end of the base sequence with the SEQ ID NO description;
1 µM of the second primer (SEQ ID NO is listed in Table 1);
20 nM of the intercalating fluorescent dye-labeled nucleic acid probe (SEQ ID NO is listed in Table 1): this was prepared in Example 2;
0.16 µM of the oligonucleotide for cleavage (SEQ ID NO is listed in Table 1): the 3' end-OH was modified by amino group;
6 U/30 µl of ribonuclease inhibitor (manufactured by Takara Bio);
13% DMSO.

(3) After the reaction solution was kept at 43°C for 5 minutes, 5 µl of enzyme solution, which was kept at 43 °C for 2 minutes beforehand, was added as the following composition.
[The composition of enzyme solution: the final concentration (in 30 µl)]
2% sorbitol;
6.4 U/30 µl of AMV reverse transcriptase (manufactured by Life Science);
142 U/30 µl of T7 RNA polymerase (manufactured by Invitrogen);
3.6 µg/30 µl of bovine serum albumin.

(4) Subsequently, by using a fluorescence spectrophotometer featured with temperature control that is capable of directly measuring PCR tubes, the fluorescence intensity of the reaction solution (excitation wavelength 470 nm, fluorescence wavelength 520 nm) was measured during the reaction at 43°C. The time of enzyme addition was set at 0 minute. The case where the ratio of fluorescence intensity of the reaction solution exceeded 1.2, was determined to be (+), and the results that defined the time at the determination as detection time were shown in Table 1.

By using a combination of the first primer, the second primer, the intercalating fluorescent dye-labeled nucleic acid probe, and the oligonucleotide for cleavage shown in Table 1, 10⁶, 10³, 10² or 50 copies/test of 16S ribosomal RNA of *Legionella pneumophila* were detected within 30 minutes.

In other words, by using a combination of the sequence selected from SEQ ID NO: 5 and 12-18 as the first primer, the sequence selected from SEQ ID NO: 4, 19-29 and 45 as the second primer, the sequence selected from SEQ ID NO: 6 and 44 as the intercalating fluorescent dye-labeled nucleic acid probe, and the sequence selected from SEQ ID NO: 30-35 as the oligonucleotide for cleavage, or a combination of the sequence selected from SEQ ID NO: 8, 36, and 37 as the first primer, the sequence selected from SEQ ID NO: 38-42 as the second primer, the sequence shown in SEQ ID NO: 11 as the intercalating fluorescent dye-labeled nucleic acid probe, and the sequence shown in SEQ ID NO: 43 as the oligonucleotide for cleavage, in each case, 16S ribosomal RNA of *Legionella pneumophila* was detected quickly.

Here, each SEQ ID NO: 5 and 12-18 is a partial sequence of SEQ ID NO: 50. Each SEQ ID NO: 4, 19-29, and 45 is a partial sequence of SEQ ID NO: 3. Each SEQ ID NO: 36 and 37 is a partial sequence of SEQ ID NO: 10. Each SEQ ID NO: 38-42 is a partial sequence of SEQ ID NO: 9.

Thus, by using a primer set containing the second primer having at least 15 nucleotides of SEQ ID NO: 3 and the first primer having at least 15 nucleotides of SEQ ID NO: 50, and a primer set containing the second primer having at least 15 nucleotides of SEQ ID NO: 9 and the first primer having at least 15 nucleotides of SEQ ID NO: 10, it was shown that ribosomal RNA of a bacterium belonging to the genus *Legionella* was detected quickly with high sensitivity.

By the RNA amplification and measurement method using the first primer, the second primer, and the intercalating fluorescent dye-labeled nucleic acid probe of the invention, these described above showed that 16S ribosomal RNA of *Legionella pneumophila* was detected quickly.

**[Table 1]**

| Combination of oligonucleotides | | | | Measurement | |
|---|---|---|---|---|---|
| First primer (SEQ ID NO) | Second primer (SEQ ID NO) | Intercalating fluorescent dye-labeled Fluorescent probe (SEQ ID NO) | Oligonucleotide for cleavage (SEQ ID NO) | 10^6 copy Detection time | Determi nation |
| 13 | 20 | 6 | 31 | 14.24 | (+) |
| 5 | 26 | 6 | 31 | 9,76 | (+) |
| 5 | 20 | 6 | 31 | 14.77 | (+) |
| 36 | 42 | 11 | 43 | 13.73 | (+) |
| 36 | 41 | 11 | 43 | 14.58 | (+) |
| 36 | 39 | 11 | 43 | 14.60 | (+) |
| 36 | 38 | 11 | 43 | 24.96 | (+) |
| 36 | 40 | 11 | 43 | 25.43 | (+) |
| 37 | 39 | 11 | 43 | 16.67 | (+) |
| 37 | 38 | 11 | 43 | 20.66 | (+) |
| 8 | 42 | 11 | 43 | 15.42 | (+) |
| 8 | 41 | 11 | 43 | 17.53 | (+) |
| 8 | 39 | 11 | 43 | 19.91 | (+) |
| 14 | 21 | 6 | 31 | 10.59 | (+) |
| 14 | 20 | 6 | 31 | 15.27 | (+) |

**[Table 2]**

| Combination of oligonucleotides | | | | Measurement | |
|---|---|---|---|---|---|
| First primer (SEQ ID NO) | Second primer (SEQ ID NO) | intercalating fluorescent dye-labeled Fluorescent probe (SEQ ID NO) | Oligonucleotide for cleavage (SEQ ID NO) | Measurement 10^3copy Detection time | Determination |
| 12 | 20 | 6 | 30 | 18.82 | (+) |
| 13 | 21 | 6 | 31 | 14.98 | (+) |
| 13 | 24 | 6 | 31 | 16.28 | (+) |
| 13 | 22 | 6 | 31 | 23.93 | (+) |
| 5 | 21 | 6 | 35 | 12.93 | (+) |
| 5 | 21 | 6 | 31 | 15.51 | (+) |
| 5 | 19 | 44 | 31 | 18.80 | (+) |
| 16 | 21 | 6 | 33 | 25.76 | (+) |
| 17 | 21 | 6 | 35 | 27.10 | (+) |
| 11 | 21 | 6 | 34 | 12.71 | (+) |
| 14 | 23 | 6 | 31 | 12.76 | (+) |
| 15 | 21 | 6 | 32 | 20.42 | (+) |
| 18 | 4 | 6 | 35 | 11.64 | (+) |
| 18 | 45 | 6 | 35 | 11.46 | (+) |
| 18 | 19 | 6 | 35 | 11.71 | (+) |
| 18 | 27 | 6 | 35 | 13.57 | (+) |
| 18 | 26 | 6 | 35 | 20.41 | (+) |
| 18 | 21 | 6 | 35 | 16.39 | (+) |

**[Table 3]**

| Combination of oligonucleotides | | | | Measurement | |
|---|---|---|---|---|---|
| First primer (SEQ ID NO) | Second primer (SEQ ID NO) | Intercalating fluorescent dye-labeled Fluorescent probe (SEQ ID NO) | Oligonucleotide for cleavage (SEQ ID NO) | Measurement 10^2copy Detection time | Determination |
| 13 | 4 | 6 | 31 | 14.45 | (+) |
| 13 | 23 | 6 | 31 | 15.54 | (+) |
| 13 | 25 | 6 | 31 | 18.94 | (+) |
| 5 | 29 | 6 | 31 | 12.86 | (+) |
| 5 | 4 | 6 | 31 | 11.96 | (+) |
| 18 | 19 | 6 | 35 | 17.39 | (+) |

**[Table 4]**

| Combination of oligonucleotides | | | | Measurement | |
|---|---|---|---|---|---|
| First primer (SEQ ID NO) | Second primer (SEQ ID NO) | labeled Fluorescent probe (SEQ ID NO) | Intercalating fluorescent dye-Oligonucleotide for Measurement cleavage (SEQ ID NO) | 50copy Detection time | Determination |
| 13 | 19 | 6 | 31 | 16.87 | (+) |
| 5 | 28 | 6 | 31 | 13.00 | (+) |
| 5 | 27 | 6 | 31 | 14.33 | (+) |
| 36 | 42 | 11 | 43 | 20.50 | (+) |
| 36 | 39 | 11 | 43 | 29.10 | (+) |

In respect to Tables 2-4, by using the combination of the oligonucleotides in the above Table, the RNA amplification and fluorescence measurement of 50, 10², 10³, 10⁶ copies/test of 16S ribosomal RNA of *Legionella pneumophila* as a sample were performed. The case where the ratio of fluorescence intensity exceeded 1.2, was determined to be (+), the time at the determination was defined as detection time.

### Example 4

[Sample (a bacterium belonging to the genus *Legionella*)] (1) The seven species and seven strains belonging to the genus *Legionella* shown in Table 5 were grown in BCYEα agar media. After each of the growing colonies of bacteria was obtained with a sterile swab, the bacteria were suspended in TE buffer (10 mM Tris-HCl, 1 mM EDTA, pH 8.0), and were diluted to about 100 cfu/50 µl. Then, RNA was extracted.

(2) 20 µl of reaction solution of the following composition was aliquoted in 0.5 ml volume PCR tubes (Gene Amp Thin-Walled Reaction Tubes, manufactured by Perkin Elmer), and 5µl of the extracted RNA sample was added thereto.
[The composition of reaction solution: concentration is the final concentration after addition of enzyme solution (in 30 µl)]
60 mM Tris· HCl (pH 8.6);
18 mM magnesium chloride;
100 mM potassium chloride;
1 mM DTT;
Each 0.25 mM dATP, dCTP, dGTP, dTTP;
Each 3 mM ATP, CTP, UTP, GTP;
3.6 mM ITP;
1 µM of the first primer (SEQ ID NO: 5): T7 polymerase promoter sequence (SEQ ID NO: 46) was added to the 5' end of the base sequence with the SEQ ID NO description;
1 µM of the second primer (SEQ ID NO is listed in Table 5);
20 nM of the intercalating fluorescent dye-labeled nucleic acid probe (SEQ ID NO: 6): this was prepared in Example 2;
0.16 µM of the oligonucleotide for cleavage (SEQ ID NO: 31): the 3' end-OH was modified by amino group;
6 U/30 µl of ribonuclease inhibitor (manufactured by Takara Bio);
13% DMSO.

(3) After the reaction solution was kept at 43°C for 5 minutes, 5µl of enzyme solution, which was kept at 43°C for 2 minutes beforehand, was added as the following composition.
[The composition of enzyme solution: the final concentration (in 30 µl)]
2% sorbitol;
6.4 U/30 µl of AMV reverse transcriptase (manufactured by Life Science);
142 U/30 µl of T7 RNA polymerase (manufactured by Invitrogen);
3.6 µg/30 µl of bovine serum albumin.

(4) Subsequently, by using a fluorescence spectrophotometer featured with temperature control that is capable of directly measuring PCR tubes, the fluorescence intensity of the reaction solution (excitation wavelength 470 nm, fluorescence wavelength 520 nm) was measured during the reaction at 43°C. The time of enzyme addition was set at 0 minute. The case where the ratio of fluorescence intensity of the reaction solution exceeded 1.2 within 30 minutes, was determined to be (+), and the results were shown in Table 5.
By using the primer set shown in Table 5, all the seven species and seven strains belonging to the genus *Legionella* were detected.

**[Table 5]**

| Genus and Species | Strain NO ATCC | Cell number [cfu/test] | Measurement | |
|---|---|---|---|---|
| | | | Primer set (SEQ ID NO) | |
| | | | First primer : 5 | First primer : 5 |
| | | | Second primer: 19 | Second primer : 4 |
| | | | intercalating fluorescent dye-labeled fluorescent probe : 6 | Intercalating fluorescent dye-labeled fluorescent probe: 6 |
| | | | Oligonucleotide for cleavage: 31 | Oligonucleotide for cleavage: 31 |
| Legionella pneumophila | 33152 | 0.6 | + | + |
| Fluoribacter bozemanae | 33217 | 3.18 | + | + |
| Tatlockia micdadei | 33218 | 25.7 | + | + |
| Fluoribacter dumoffil | 33279 | 0.75 | + | + |
| Fluoribacter gormanii | 33297 | 3.9 | + | + |
| Legionella oakridgensis | 337551 | 0.1 | + | + |
| Legionella longbeachae | 33462 | 2.32 | + | + |

### Example 5

[Evaluation of specificity (Effectiveness of CT substitution)] (1) The nine species and nine strains of non*-Legionella* bacteria (*Bacillus cereus, Haemophilus influenzae, Stphylococcus aureus, Streptococcus pneumoniae, Enterococcus faecalis, Pseudomonas aeruginosa, Streptococcus pyrogenes, Streptococcus agalactiae* and *Shigella boydii*) were cultured in agar media. After each of the growing colonies of bacteria was obtained with a sterile swab, the bacteria were suspended in TE buffer (10 mM Tris-HCl, 1 mM EDTA, pH 8.0), and were diluted to about 10⁸-10⁹ cfu / 50 µl. Then, RNA was extracted.

(2) 20µl of reaction solution of the following composition was aliquoted in 0.5 ml volume PCR tubes (Gene Amp Thin-Walled Reaction Tubes, manufactured by Perkin Elmer), and 5µl of the extracted RNA sample was added thereto.

[The composition of reaction solution: concentration is the final concentration after addition of enzyme solution (in 30 µl)]
60 mM Tris· HCl (pH 8.6);
18 mM magnesium chloride;
100 mM potassium chloride;
1 mM DTT;
Each 0.25 mM dATP, dCTP, dGTP, dTTP;
Each 3 mM ATP, CTP, UTP, GTP;
3.6 mM ITP;
1 µM of the first primer (SEQ ID NO: 5): T7 polymerase promoter sequence (SEQ ID NO: 46) was added to the 5' end of the base sequence with the SEQ ID NO description;
1 µM of the second primer (SEQ ID NO is listed in Table 6);
20 nM of the intercalating fluorescent dye-labeled nucleic acid probe (SEQ ID NO: 6): this was prepared in Example 2;
0.16 µM of the oligonucleotide for cleavage (SEQ ID NO: 31): the 3' end-OH was modified by amino group;
6 U/30 µl of ribonuclease inhibitor (manufactured by Takara Bio);
13% DMSO.

(3) After the reaction solution was kept at 43°C for 5 minutes, 5 µl of enzyme solution, which was kept at 43°C for 2 minutes beforehand, was added as the following composition.
[The composition of enzyme solution: the final concentration (in 30µl)]
2% sorbitol;
6.4 U/30 µl of AMV reverse transcriptase (manufactured by Life Science);
142 U/330 µl of T7 RNA polymerase (manufactured by Invitrogen);
3.6 µg/30 µl of bovine serum albumin.

(4) Subsequently, by using a fluorescence spectrophotometer featured with temperature control that is capable of directly measuring PCR tubes, the fluorescence intensity of the reaction solution (excitation wavelength 470 nm, fluorescence wavelength 520 nm) was measured during the reaction at 43 °C. The time of enzyme addition was set at 0 minute. The case where the ratio of fluorescence intensity of the reaction solution exceeded 1.2 within 30 minutes, was determined to be (+), and the results were shown in Table 6.

**[Table 6]**

| Genus and Species | Strain NO | Cell number [cfu/test] | Measurement | |
|---|---|---|---|---|
| | | | Primer set (SEQ ID NO) | |
| | | | First primer : 5 | First primer : 5 |
| | | | Second primer : 19 | Second primer : 4 |
| | | | Intercalating fluorescent dye-labeled fluorescent probe : 6 | Intercalating fluorescent dye-labeled fluorescent probe: 6 |
| | | | Oligonucleotide for cleavage 31 | Oligonucleotide for cleavage: 31 |
| Bacillus cereus | IID 1681 | 1 × 10⁸ | - | - |
| Haemophilus influenzae | IID983 | 1 × 10⁸ | + | - |
| Staphyloooccus aureus | IID1677 | 1 × 10⁷ | + | - |
| Streptococcus pneumoniae | IID554 | 1 × 10⁸ | - | - |
| Enterococcus faecalis | RIMD3116001 | 1 × 10⁸ | + | - |
| Pseudomonas aeruginosa | GTC81 | 1 × 10⁸ | + | - |
| Streptococcus pyrogenes | GTC262 | 4 × 10⁷ | + | - |
| Streptococcus agalactiae | GTC1234 | 5 × 10⁷ | - | - |
| Shigella boydii | IID627 | 1 × 10⁸ | - | - |

By substituting cytosine to thymine in the primer sets shown in the above Table, the detection of non*-Legionella* bacteria was reduced. By using the primer set containing the oligonucleotide of SEQ ID NO: 5 as the first primer and the oligonucleotide of SEQ ID NO: 4 as the second primer, the method made it possible to increase the specificity for non*-Legionella* bacteria while maintaining the sensitivity to a bacterium belonging to the genus *Legionella* with this primer set.

### Example 6

[Specificity for viable cell (chlorine addition experiment)] (1) *Legionella pneumophila* was cultured in BCYEα agar media. After each of the growing colonies of bacteria was obtained with a sterile swab, the bacteria were suspended in TE buffer (10 mM Tris-HCl, 1 mM EDTA, pH 8.0), and were diluted to about 10⁵ cfu/50 µl. By adding hypochlorous acid to 2 mg/l of the residual chlorine concentration thereto, the detection of the number of viable cells and 16S ribosomal RNA during the elapsed time since the addition was conducted. The number of viable cells was determined by culturing in BCYEα agar media. For the detection of 16S ribosomal RNA, RNA was also extracted.

(2) 20 µl of reaction solution of the following composition was aliquoted in 0.5 ml volume PCR tubes (Gene Amp Thin-Walled Reaction Tubes, manufactured by Perkin Elmer), and 5 µl of the extracted RNA sample was added thereto.

[The composition of reaction solution: concentration is the final concentration after addition of enzyme solution (in 30 µl)]
60 mM Tris· HCl (pH 8.6);
18 mM magnesium chloride;
100 mM potassium chloride;
1 mM DTT;
Each 0.25 mM dATP, dCTP, dGTP, dTTP;
Each 3 mM ATP, CTP, UTP, GTP;
3.6 mM ITP;
1 µM of the first primer (SEQ ID NO: 5): T7 polymerase promoter sequence (SEQ ID NO: 46) was added to the 5' end of the base sequence with the SEQ ID NO description;
1 µM of the second primer (SEQ ID NO: 4);
20 nM of the intercalating fluorescent dye-labeled nucleic acid probe (SEQ ID NO: 6): this was prepared in Example 2;
0.16 µM of the oligonucleotide for cleavage (SEQ ID NO: 31): the 3' end-OH was modified by amino group;
6 U/30 µl of ribonuclease inhibitor (manufactured by Takara Bio);
13% DMSO.

(3) After the reaction solution was kept at 43°C for 5 minutes, 5 µl of enzyme solution, which was kept at 43°C for 2 minutes beforehand, was added as the following composition.
[The composition of enzyme solution: the final concentration (in 30 µl)]
2% sorbitol;
6.4 U/30 µl of AMV reverse transcriptase (manufactured by Life Science);
142 U/30 µl of T7 RNA polymerase (manufactured by Invitrogen);
3.6 µg/30 µl of bovine serum albumin.

(4) Subsequently, by using a fluorescence spectrophotometer featured with temperature control that is capable of directly measuring PCR tubes, the fluorescence intensity of the reaction solution (excitation wavelength 470 nm, fluorescence wavelength 520 nm) was measured during the reaction at 43°C. The time of enzyme addition was set at 0 minute. The case where the ratio of fluorescence intensity of the reaction solution exceeded 1.2, was determined to be (+), and the results were shown in Table 7.

(5) By way of comparison, the detection of 16S ribosomal DNA was performed using a DNA amplification kit (manufactured by Eiken Kagaku). The results were shown in Table 7. Specificity for viable and dead cells being sterilized by hypochlorous acid was compared between 16S ribosomal RNA and 16S ribosomal DNA in the detection. Although 16S ribosomal RNA was unable to be detected when the bacteria were killed, 16S ribosomal DNA was determined to be positive even 43 hours after the death of the bacteria. Based on the above results, by targeting 16S ribosomal RNA, the specific detection of viable cells became possible in comparison with 16S ribosomal DNA as a target.

**[Table 7]**

| Elapsed time [hour] | Ribosomal RNA | Ribosomal DNA | Cell number [cfu/50µl] |
|---|---|---|---|
| 0 | + | + | 10^5 |
| 0.5 | + | + | <1 |
| 1.5 | - | + | <1 |
| 3.0 | - | + | <1 |
| 43 | - | + | <1 |

### Example 7

In the method of the invention, 16S ribosomal RNA of a bacterium belonging to the genus *Legionella* was measured using various combinations of the first primer, the second primer, the intercalating fluorescent dye-labeled nucleic acid probe, and the oligonucleotide for cleavage.

(1) The 16S ribosomal RNA of *Legionella pneumophila* (including the base number 1-1397) prepared in Example 1, was diluted to 10³ copies/5 µl using the RNA diluent (10 mM Tris· HCl (pH 8.0), 1 mM EDTA, 0.25 U/µl ribonuclease inhibitor, and 5.0 mM DTT), and was used as an RNA sample.
(2) 20 µl of reaction solution of the following composition was aliquoted in 0.5 ml volume PCR tubes (Gene Amp Thin-Walled Reaction Tubes, manufactured by Perkin Elmer), and 5 µl of the RNA sample was added thereto.

[The composition of reaction solution: concentration is the final concentration after addition of enzyme solution (in 30 µl)]
60 mM Tris· HCl (pH 8.6);
18 mM magnesium chloride;
100 mM potassium chloride;
1 mM DTT;
Each 0.25 mM dATP, dCTP, dGTP, dTTP;
Each 3 mM ATP, CTP, UTP, GTP;
3.6 mM ITP;
1 µM of the first primer (SEQ ID NO is listed in Table 8): T7 polymerase promoter sequence (SEQ ID NO: 46) was added to the 5' end of the base sequence with the SEQ ID NO description;
1 µM of the second primer (SEQ ID NO is listed in Table 8);
20 nM of the intercalating fluorescent dye-labeled nucleic acid probe (SEQ ID NO is listed in Table 8): this was prepared in Example 2;
0.16 µM of the oligonucleotide for cleavage (SEQ ID NO is listed in Table 8): the 3' end-OH was modified by amino group;
6 U/30 µl of ribonuclease inhibitor (manufactured by Takara Bio);
13% DMSO.

(3) After the reaction solution was kept at 43°C for 5 minutes, 5 µl of enzyme solution, which was kept at 43°C for 2 minutes beforehand, was added as the following composition.
[The composition of enzyme solution: the final concentration (in 30 µl)]
2% sorbitol;
6.4 U/30 µl of AMV reverse transcriptase (manufactured by Life Science);
142 U/30 µl of T7 RNA polymerase (manufactured by Invitrogen);
3.6 µg/30 µl of bovine serum albumin.

(4) Subsequently, by using a fluorescence spectrophotometer featured with temperature control that is capable of directly measuring PCR tubes, the fluorescence intensity of the reaction solution (excitation wavelength 470 nm, fluorescence wavelength 520 nm) was measured during the reaction at 43°C. The time of enzyme addition was set at 0 minute. The case where the ratio of fluorescence intensity of the reaction solution exceeded 1.2, was determined to be (+), and the results that defined the time at the determination as detection time were shown in Table 8.

By using the combination of the first primer, the second primer, the intercalating fluorescent dye-labeled nucleic acid probe, and the oligonucleotide for cleavage shown in Table 8, 10² copies/test of 16S ribosomal RNA of *Legionella pneumophila* were detected within 30 minutes.

In other words, by using the combination of the sequence selected from SEQ ID NO: 51-54 as the first primer, the sequence selected from SEQ ID NO: 4 and 45 as the second primer, the sequence of SEQ ID NO: 6 as the intercalating fluorescent dye-labeled nucleic acid probe, and the sequence selected from SEQ ID NO: 31 and 35 as the oligonucleotide for cleavage, in each case, 16S ribosomal RNA of *Legionella pneumophila* was detected quickly.

Here, each SEQ ID NO: 51-54 is a partial sequence of SEQ ID NO: 2. Each SEQ ID NO: 4 and 45 is a partial sequence of SEQ ID NO: 3.

Thus, by using the primer set containing the second primer having at least 15 nucleotides of SEQ ID NO: 3 and the first primer having at least 15 nucleotides of SEQ ID NO: 2, it was shown that ribosomal RNA of a bacterium belonging to the genus *Legionella* was detected quickly with high sensitivity.

By the RNA amplification and measurement method using the first primer, the second primer, and the intercalating fluorescent dye-labeled nucleic acid probe of the invention, these described above showed that 16S ribosomal RNA of *Legionella pneumophila* was detected quickly.

**[Table 8]**

| Combination of oligonucleotides | | | | Measurement | |
|---|---|---|---|---|---|
| First primer (SEQ ID NO) | Second primer (SEQ ID NO) | Intercalating fluorescent dye-labeled Fluorescent probe (SEQ ID NO) | Oligonucleotide for cleavage (SEQ ID NO) | Measurement 10^2copy Detection time | Determination |
| 51 | 45 | 6 | 55 | 12.10 | (+) |
| 52 | 45 | 6 | 31 | 13.38 | (+) |
| 53 | 45 | 6 | 31 | 17.21 | (+) |
| 54 | 4 | 6 | 31 | 12.78 | (+) |
| 54 | 45 | 6 | 31 | 11.96 | (+) |

### Example 8

[Detection sensitivity] In the method of the invention, the detection sensitivity of 16S ribosomal RNA of a bacterium belonging to the genus *Legionella* was measured using the combination of the first primer, the second primer, the intercalating fluorescent dye-labeled nucleic acid probe, and the oligonucleotide for cleavage shown in Table 9.

(1) The 16S ribosomal RNA of *Legionella pneumophila* (including the base number 1-1397) prepared in Example 1, was diluted to 10³, 10², 50, or 30 copies/5 µl using the RNA diluent (10 mM Tris· HCl (pH 8.0), 1 mM EDTA, 0.25 U/µl ribonuclease inhibitor, and 5.0 mM DTT), and was used as an RNA sample. (2) 20 µl of reaction solution of the following composition was aliquoted in 0.5 ml volume PCR tubes (Gene Amp Thin-Walled Reaction Tubes, manufactured by Perkin Elmer), and 5 µl of the RNA sample was added thereto.

[The composition of reaction solution: concentration is the final concentration after addition of enzyme solution (in 30 µl)]
60 mM Tris· HCl (pH 8.6);
18 mM magnesium chloride;
100 mM potassium chloride;
1 mM DTT;
Each 0.25 mM dATP, dCTP, dGTP, dTTP;
Each 3 mM ATP, CTP, UTP, GTP;
3.6 mM ITP;
1 µM of the first primer (SEQ ID NO is listed in Table 9): T7 polymerase promoter sequence (SEQ ID NO: 46) was added to the 5' end of the base sequence with the SEQ ID NO description;
1 µM of the second primer (SEQ ID NO is listed in Table 9);
20 nM of intercalating fluorescent dye-labeled nucleic acid probe (SEQ ID NO is listed in Table 9): this was prepared in Example 2;
0.16 µM of the oligonucleotide for cleavage (SEQ ID NO is listed in Table 9): the 3' end-OH was modified by amino group;
6 U/30 µl of ribonuclease inhibitor (manufactured by Takara Bio);
13% DMSO.

(3) After the reaction solution was kept at 43°C for 5 minutes, 5 µl of enzyme solution, which was kept at 43°C for 2 minutes beforehand, was added as the following composition.
[The composition of enzyme solution: the final concentration (in 30 µl)]
2% sorbitol;
6.4 U/30 µl ofAMV reverse transcriptase (manufactured by Life Science);
142 U/30 µl of T7 RNA polymerase (manufactured by Invitrogen);
3.6 µg/30 µl of bovine serum albumin.

(4) Subsequently, by using a fluorescence spectrophotometer featured with temperature control that is capable of directly measuring PCR tubes, the fluorescence intensity of the reaction solution (excitation wavelength 470 nm, fluorescence wavelength 520 nm) was measured during the reaction at 43°C. The time of enzyme addition was set at 0 minute. The case where the ratio of fluorescence intensity of the reaction solution exceeded 1.2 within 20 minutes, was determined to be (+), and the results were shown in Table 9.

By using the combination of the first primer, the second primer, the intercalating fluorescent dye-labeled nucleic acid probe, and the oligonucleotide for cleavage shown in Table 9, 10³ copies/test of 16S ribosomal RNA of *Legionella pneumophila* were detected within 20 minutes.

Furthermore, by using the combination of the sequence selected from SEQ ID NO: 51 as the first primer, the sequence selected from SEQ ID NO: 45 as the second primer, the sequence of SEQ ID NO: 6 as the intercalating fluorescent dye-labeled nucleic acid probe, and the sequence selected from SEQ ID NO: 55 as the oligonucleotide for cleavage, 30 copies/test of 16S ribosomal RNA of *Legionella pneumophila* was detected within 20 minutes.

By the RNA amplification and measurement method using the first primer, the second primer, and the intercalating fluorescent dye-labeled nucleic acid probe of the invention, these described above showed that 16S ribosomal RNA of *Legionella pneumophila* was detected even in the low-copy number case.

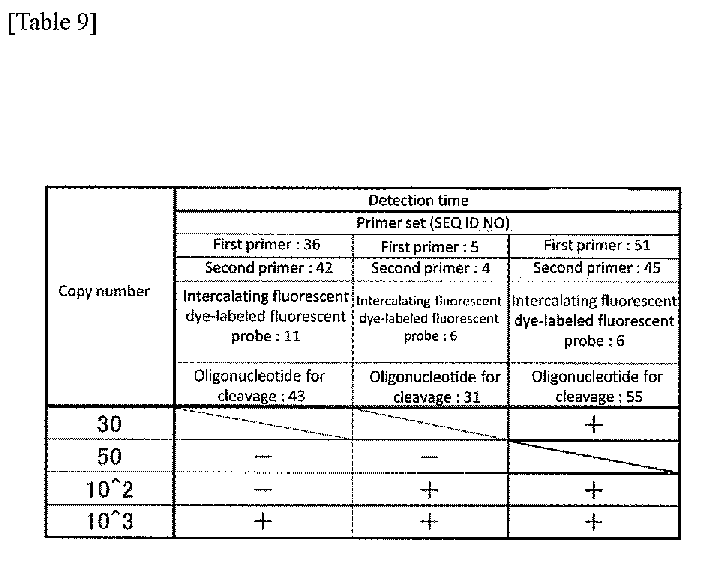

### Example 9

[Sample (a bacterium belonging to the genus *Legionella)]* (1) The seven species and seven strains belonging to the genus *Legionella* shown in Table 10 were grown in BCYEα agar media. After each of the growing colonies of bacteria was obtained with a sterile swab, the bacteria were suspended in TE buffer (10 mM Tris-HCl, 1 mM EDTA, pH 8.0), and were diluted to about 100 cfu/50 µl. Then, RNA was extracted.

(2) 20 µl of reaction solution of the following composition was aliquoted in 0.5 ml volume PCR tubes (Gene Amp Thin-Walled Reaction Tubes, manufactured by Perkin Elmer), and 5 µl of the extracted RNA sample was added thereto.
[The composition of reaction solution: concentration is the final concentration after addition of enzyme solution (in 30 µl)]
60 mM Tris· HCl (pH 8.6);
18 mM magnesium chloride;
100 mM potassium chloride;
1 mM DTT;
Each 0.25 mM dATP, dCTP, dGTP, dTTP;
Each 3 mM ATP, CTP, UTP, GTP;
3.6 mM ITP;
1 µM of the first primer (SEQ ID NO: 51): T7 polymerase promoter sequence (SEQ ID NO: 46) was added to the 5' end of the base sequence with the SEQ ID NO description;
1 µM of the second primer (SEQ ID NO: 45);
20 nM of the intercalating fluorescent dye-labeled nucleic acid probe (SEQ ID NO: 6): this was prepared in Example 2;
0.16 µM of the oligonucleotide for cleavage (SEQ ID NO: 55): the 3' end-OH was modified by amino group;
6 U/30 µl of ribonuclease inhibitor (manufactured by Takara Bio);
13% DMSO.

(3) After the reaction solution was kept at 43°C for 5 minutes, 5 µl of enzyme solution, which was kept at 43°C for 2 minutes beforehand, was added as the following composition.
[The composition of enzyme solution: the final concentration (in 30 µl)]
2% sorbitol;
6.4 U/30 µl of AMV reverse transcriptase (manufactured by Life Science);
142 U/30 µl of T7 RNA polymerase (manufactured by Invitrogen);
3.6 µg/30 µl of bovine serum albumin.

(4) Subsequently, by using a fluorescence spectrophotometer featured with temperature control that is capable of directly measuring PCR tubes, the fluorescence intensity of the reaction solution (excitation wavelength 470 nm, fluorescence wavelength 520 nm) was measured during the reaction at 43°C. The time of enzyme addition was set at 0 minute. The case where the ratio of fluorescence intensity of the reaction solution exceeded 1.2 within 30 minutes, was determined to be (+), and the results were shown in Table 10.
By using the primer set shown in Table 10, all the seven species and seven strains belonging to the genus *Legionella* were detected.

**[Table 10]**

| Genus and Species | Strain NO ATCC | Cell number [cfu/test] | Measurement |
|---|---|---|---|
| | | | Primer set (SEQ ID NO) |
| | | | First primer : 51 |
| | | | Second primer : 45 |
| | | | Intercalating fluorescent dye-labeled fluorescent probe : 6 |
| | | | Oligonucleotide for cleavage : 55 |
| Legionella pneumophila | 33152 | 0.03 | + |
| Fluoribacter bozemanae | 33217 | 1.0 | + |
| Tatlockia micdadei | 33218 | 0.1 | + |
| Fluoribacter dumoffii | 33279 | 17.5 | + |
| Fluoribacter gormanii | 33297 | 2.0 | + |
| Legionella oakridgensis | 33761 | 1.5 | + |
| Legionella longbeachae | 33462 | 1.5 | + |

### Example 10

[Evaluation of specificity] (1) The nine species and nine strains of non-*Legionella* bacteria *(Bacillus cereus, Haemophilus influenzae, Stphylococcus aureus, Streptococcus pneumoniae, Enterococcus faecalis, Pseudomonas aeruginosa, Streptococcus pyrogenes , Streptococcus agalactiae,* and *Shigella boydii*) were cultured in agar media. After each of the growing colonies of bacteria was obtained with a sterile swab, the bacteria were suspended in TE buffer (10 mM Tris-HCl, 1 mM EDTA, pH 8.0), and were diluted to about 10⁷ cfu/50 µl. Then, RNA was extracted.

(2) 20 µl of reaction solution of the following composition was aliquoted in 0.5 ml volume PCR tubes (Gene Amp Thin-Walled Reaction Tubes, manufactured by Perkin Elmer), and 5 µl of the extracted RNA sample was added thereto.

[The composition of reaction solution: concentration is the final concentration after addition of enzyme solution (in 30 µl)]
60 mM Tris· HCl (pH 8.6);
18 mM magnesium chloride;
100 mM potassium chloride;
1 mM DTT;
Each 0.25 mM dATP, dCTP, dGTP, dTTP;
Each 3 mM ATP, CTP, UTP, GTP;
3.6 mM ITP;
1 µM of the first primer (SEQ ID NO: 51): T7 polymerase promoter sequence (SEQ ID NO: 46) was added to the 5' end of the base sequence with the SEQ ID NO description;
1 µM of the second primer (SEQ ID NO: 45);
20 nM of the intercalating fluorescent dye-labeled nucleic acid probe (SEQ ID NO: 6): this was prepared in Example 2;
0.16 µM of the oligonucleotide for cleavage (SEQ ID NO: 55): the 3' end-OH was modified by amino group;
6 U/30 µl of ribonuclease inhibitor (manufactured by Takara Bio);
13% DMSO.

(3) After the reaction solution was kept at 43°C for 5 minutes, 5 µl of enzyme solution, which was kept at 43°C for 2 minutes beforehand, was added as the following composition.
[The composition of enzyme solution: the final concentration (in 30 µl)]
2% sorbitol;
6.4 U/30 µl of AMV reverse transcriptase (manufactured by Life Science);
142 U/30 µl of T7 RNA polymerase (manufactured by Invitrogen);
3.6 µg/30 µl of bovine serum albumin.

(4) Subsequently, by using a fluorescence spectrophotometer featured with temperature control that is capable of directly measuring PCR tubes, the fluorescence intensity of the reaction solution (excitation wavelength 470 nm, fluorescence wavelength 520 nm) was measured during the reaction at 43°C. The time of enzyme addition was set at 0 minute. The case where the ratio of fluorescence intensity of the reaction solution exceeded 1.2 within 30 minutes, was determined to be (+), and the results were shown in Table 11.

**[Table 11]**

| Genus and Species | Strain NO | Cell number [cfu/test] | Measurement |
|---|---|---|---|
| | | | Primer set (SEQ ID NO) |
| | | | First primer : 51 |
| | | | Second primer : 45 |
| | | | Intercalating fluorescent dye-labeled fluorescent probe : 6 |
| | | | Oligonucleotide for cleavage : 55 |
| Bacillus cereus | IID 1681 | 1 × 10⁶ | - |
| Haemophilus influenzae | IID983 | 1 × 10⁶ | - |
| Staphylococcus aureus | IID1677 | 1 × 10⁶ | - |
| Streptococcus pneumoniae | IID554 | 1 × 10⁶ | - |
| Enterococcus faecalis | RIMD3116001 | 1 × 10⁶ | - |
| Pseudomonas aeruginosa | GTC81 | 1 × 10⁶ | - |
| Streptococcus pyrogenes | GTC262 | 1 × 10⁶ | - |
| Streptococcus agalactiae | GTC1234 | 1 × 10⁶ | - |
| Shigella boydii | IID627 | 1 × 10⁶ | - |

Among the primer set shown in the above Table, by using the primer set containing the oligonucleotide of SEQ ID NO: 51 as the first primer and the oligonucleotide of SEQ ID NO: 45 as the second primer, the method made it possible to increase the specificity for non-*Legionella* bacteria while maintaining the sensitivity to a bacterium belonging to the genus *Legionella* with this primer set.

The above experiments of Example 1-10, by using the primers and primer sets of the invention, demonstrated that ribosomal RNA of a bacterium belonging to the genus *Legionella* was detected with high specificity for viable cells, quickly, and with high sensitivity.

### INDUSTRIAL APPLICABILITY

The aspects of the present invention are useful in medicine, pharmacology, microbiology, and areas of public health such as water quality managements of a public bath, a swimming pool, environmental water, cooling water of buildings, and the like. These fields require the detection of a bacterium belonging to the genus *Legionella*.

## Claims

1. A primer set capable of amplifying nucleic acid using ribosomal RNA of a bacterium belonging to the genus *Legionella* as a target, comprising an antisense primer containing nucleotides of at least 15 or more bases hybridizing specifically with a nucleic acid of a base sequence of SEQ ID NO: 1 or 7 under stringent conditions, and a sense primer containing nucleotides of at least 15 or more bases hybridizing specifically with a nucleic acid of a base sequence of SEQ ID NO: 47 or 49 under stringent conditions.

2. A primer set capable of amplifying nucleic acid using ribosomal RNA of a bacterium belonging to the genus *Legionella* as a target, comprising an antisense primer containing nucleotides of at least 15 or more bases having 80% or more homology with a portion of a base sequence of SEQ ID NO: 45 or 48, and a sense primer containing nucleotides of at least 15 or more bases having 80% or more homology with a portion of a base sequence of SEQ ID NO: 2 or 8.

3. The primer set of claim 1 or 2, wherein the antisense primer contains at least 15 or more nucleotides within a base sequence of SEQ ID NO: 3 or 9.

4. The primer set of claim 3, wherein the antisense primer contains nucleotides of a base sequence of SEQ ID NO: 4.

5. The primer set of claim 1 or 2, wherein the sense primer contains at least 15 or more nucleotides within a base sequence of SEQ ID NO: 50 or 10.

6. The primer set of claim 1 or 2, wherein the sense primer contains at least 15 or more nucleotides within a base sequence of SEQ ID NO: 5.

7. The primer set of claim 1 or 2, wherein at least one of the antisense and the sense primers further comprises a sequence which plays a role in specific functions such as a promoter sequence of RNA polymerase, a sequence constituting a stem-loop structure, a restriction enzyme site, and the like.

8. A method for detecting ribosomal RNA of a bacterium belonging to the genus *Legionella,* comprising the steps of:
obtaining amplified products by performing a nucleic acid amplification using the primer set of claim 1 or 2 and ribosomal RNA of a bacterium belonging to the genus *Legionella* as a template, and simultaneously or subsequently to the previous step; and
detecting the amplified products using a nucleic acid probe containing nucleotides of at least 15 bases having 80% or more homology with either a base sequence shown in SEQ ID NO: 6 or 11 or a complementary base sequence thereof labeled by means for generating a detectable signal.

9. A detection kit of ribosomal RNA of a bacterium belonging to the genus *Legionella* comprising the primer set of claim 1 or 2 in order to obtain amplified products by performing a nucleic acid amplification using ribosomal RNA of a bacterium belonging to the genus *Legionella* as a template; and
a nucleic acid probe containing at least 15 nucleotides having 80% or more homology with either a base sequence shown in SEQ ID NO: 6 or 11 or a complementary base sequence thereof labeled by means for generating a detectable signal to detect the amplified products.

10. The method of claim 8, wherein the step of amplifying nucleic acid and obtaining amplified products comprises the steps of:
(1) producing double-stranded DNA comprising a promoter sequence and a specific base sequence downstream of the promoter sequence by a first primer homologous to at least a portion of a downstream region from a 5' end of a specific base sequence of ribosomal RNA of a bacterium belonging to the genus *Legionella* and a second primer complementary to a portion of a upstream region from a 3' end of a specific base sequence (at least one of the first and second primers has a promoter sequence at its 5' end);
(2) producing RNA transcripts using the double-stranded DNA as a template; and
(3) amplifying the RNA transcripts in a chain reaction in which the RNA transcripts continuously become a template for DNA synthesis.

11. The detection kit of claim 9 for practicing the method of claim 10, further comprising at least RNA-dependent DNA polymerase, ribonuclease H (RNase H), DNA-dependent DNA polymerase, and RNA polymerase.
